# EUROPEAN PATENT APPLICATION

(11) **EP 4 420 694 A1**
(43) Date of publication of application: **28.08.2024**
(21) Application number: 23158595.1
(22) Date of filing: 24.02.2023
(51) Int. Cl.: A61M 5/20, A61M 5/315, A61M 5/32

(54) **AUTO-INJECTOR WITH ASSEMBLY AND ALIGNMENT FEATURES**

(71) Applicant: Becton, Dickinson and Company, Franklin Lakes, NJ 07417-1880 (US)
(72) Inventor: BESSON, Nicolas, 38650 Treffort (FR); PLOUVIER, Adrien, 38400 SAINT MARTIN D'HERES (FR); ALVAIN, Olivier, 38180 SEYSSINS (FR)
(74) Representative: Germain Maureau

(57) **Abstract**

A drug delivery device includes an upper housing shell, a lower housing shell, and a syringe. The syringe includes a barrel, a stopper, and a needle, at least a portion of the syringe positioned within the lower housing shell. The drug delivery device further includes a drive assembly including a plunger body configured to move the stopper within the barrel upon actuation of the drive assembly, a cassette body configured to receive the syringe, and a motor body configured to engage the cassette body. The motor body includes a pair of sidewalls and at least one shell stop extending outward from the sidewalls and configured to engage an internal structure of the upper housing shell.

## Description

### BACKGROUND OF THE INVENTION

### Field of the Invention

The present disclosure relates generally to a drug delivery device and, more specifically, to an auto-injector.

### Description of Related Art

Various types of automatic injection devices have been developed to allow drug solutions and other liquid therapeutic preparations to be administered by untrained personnel or to be self-injected. Generally, these devices include a reservoir that is pre-filled with the liquid therapeutic preparation, and some type of automatic needle-injection mechanism that can be triggered by the user. Many of these devices, such as auto-injectors, are designed so that the reservoir, such as a pre-filled syringe, is assembled into the device during assembly of the device. In addition to automatically deploying the needle-injection mechanism, many drug delivery devices also automatically shield the needle after use of the device to prevent any unintended contact with the needle.

Automatic injection devices include several interlocking parts that must be assembled in a predetermined order for the devices to function effectively as intended. As such, there is an ongoing need in the art to minimize the number of steps required in the assembly process, as well as to guide alignment of the various components during assembly, to ensure adequate functionality of the devices.

### SUMMARY OF THE INVENTION

Provided herein is a drug delivery device which includes, in some embodiments, an upper housing shell, a lower housing shell, and a syringe. The syringe includes a barrel, a stopper, and a needle. At least a portion of the syringe positioned within the lower housing shell. The drug delivery device further includes a drive assembly including a plunger body configured to move the stopper within the barrel upon actuation of the drive assembly, a cassette body configured to receive the syringe, and a motor body configured to engage the cassette body. The motor body includes a pair of sidewalls and at least one shell stop extending outward from the sidewalls and configured to engage an internal structure of the upper housing shell.

In some embodiments, the at least one shell stop includes a planar proximal face.

In some embodiments, the at least one shell stop includes a cantilevered end.

In some embodiments, the at least one shell stop includes a radiused edge reinforcing the cantilevered end.

In some embodiments, sidewalls of the motor body are configured to deflect inward during connection of the motor body to the cassette body, and the at least one shell stop extends a sufficient distance outward from the sidewalls so that the at least one shell stop remains at least partially engaged with the internal structure of the upper housing shell when the sidewalls are deflected inward during connection of the motor body to the cassette body.

In some embodiments, the upper housing shell includes a pair of spaced apart reinforcing ribs extending along a longitudinal axis of the upper housing shell.

In some embodiments, the reinforcing ribs have an L-shaped, Z-shaped, or stepped profile.

In some embodiments, the upper housing shell includes a connecting wall extending between and connecting the reinforcing ribs.

In some embodiments, the connecting wall extends from a proximal end of the upper housing shell.

In some embodiments, the connecting wall terminates at a location between the proximal end of the upper housing shell and distal ends of the reinforcing ribs.

In some embodiments, the drug delivery device further includes a first subassembly including the lower housing shell and the cassette body; and a second subassembly including the upper housing shell and the motor body. The second subassembly is configured to be brought into engagement with the first subassembly to assemble the drug delivery device.

In some embodiments, the lower housing shell includes an inner lip, and the upper housing shell includes an outer lip configured to engage the inner lip.

Other embodiments of the present disclosure are directed to a drug delivery device including an upper housing shell, a lower housing shell, and a syringe. The syringe includes a barrel, a stopper, and a needle. At least a portion of the syringe is positioned within the lower housing shell. The drug delivery device further includes a drive assembly including a plunger body configured to move the stopper within the barrel upon actuation of the drive assembly, a cassette body configured to receive the syringe, and a motor body configured to engage the cassette body. The upper housing shell includes a pair of spaced apart reinforcing ribs extending along a longitudinal axis of the upper housing shell, and a connecting wall extending between and connecting the reinforcing ribs.

In some embodiments, the connecting wall extends from a proximal end of the upper housing shell.

In some embodiments, the connecting wall terminates at a location between the proximal end of the upper housing shell and distal ends of the reinforcing ribs.

Other embodiments of the present disclosure are directed to a method of assembling a drug delivery device. The method includes inserting a syringe into a first subassembly, the first subassembly including a cassette body, removing a safety pin from a motor body of a second subassembly, and bringing the second subassembly into engagement with the first subassembly such that at least one cassette clip of the motor body engages at least one opening of the cassette body, and an outer lip of the second subassembly engages an inner lip of the first subassembly.

In some embodiments, the method further includes applying a label to the first subassembly and the second subassembly.

In some embodiments, the motor body includes a pair of sidewalls and at least one shell stop extending outward from the sidewalls. The at least one shell stop is configured to engage an upper housing shell of the second subassembly.

In some embodiments, the at least one shell stop includes a cantilevered end and a radiused edge.

In some embodiments, an upper housing shell of the second subassembly includes a pair of spaced-apart reinforcing ribs, and a connecting wall extending between and connecting the reinforcing ribs.

These and other features and characteristics of drug delivery devices and methods of operation and functions of the related elements of structures and the combination of parts and economies of manufacture, will become more apparent upon consideration of the following description and the appended claims with reference to the accompanying drawings, all of which form a part of this specification. It is to be expressly understood, however, that the drawings are for the purpose of illustration and description only.

### BRIEF DESCRIPTION OF THE DRAWINGS

FIG. 1 is a perspective view of a drug delivery device according to non-limiting embodiments described herein;
FIG. 2 is a cross-sectional view of the drug delivery device of FIG. 1;
FIG. 3 is a perspective view of a second subassembly of the drug delivery device of FIG. 1;
FIG. 4 is a perspective view of a first subassembly of the drug delivery device of FIG. 1;
FIG. 5 is a perspective view of a motor body of the drug delivery device of FIG. 1;
FIG. 6 is a detail perspective view of the motor body of FIG. 5;
FIG. 7 is a top perspective view of a cassette body of the drug delivery device of FIG. 1;
FIG. 8 is a bottom perspective view of the cassette body of FIG. 7;
FIG. 9 is an exploded perspective view of the first subassembly and syringe of the drug delivery device of FIG. 1;
FIG. 10 is an exploded perspective view of the first subassembly and second subassembly of the drug delivery device of FIG. 1;
FIG. 11 is a side view of the assembled drug delivery device of FIG. 1; and
FIG. 12 is a cross-sectional view of an upper housing shell of the drug delivery device of FIG. 1.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

It should be understood that any numerical range recited herein is intended to include all values and sub-ranges subsumed therein. For example, a range of "1 to 10" is intended to include all sub-ranges between (and including) the recited minimum value of 1 and the recited maximum value of 10, that is, having a minimum value equal to or greater than 1 and a maximum value of equal to or less than 10.

When used in relation to a component of a medical device, the term "distal" refers to a portion of said component nearest to a patient. When used in relation to a component of a medical device, the term "proximal" refers to a portion of said component farthest from the patient.

As used herein, the term "at least one of" is synonymous with "one or more of'. For example, the phrase "at least one of A, B, and C" means any one of A, B, and C, or any combination of any two or more of A, B, and C. For example, "at least one of A, B, and C" includes one or more of A alone; or one or more of B alone; or one or more of C alone; or one or more of A and one or more of B; or one or more of A and one or more of C; or one or more of B and one or more of C; or one or more of all of A, B, and C. Similarly, as used herein, the term "at least two of' is synonymous with "two or more of'. For example, the phrase "at least two of D, E, and F" means any combination of any two or more of D, E, and F. For example, "at least two of D, E, and F" includes one or more of D and one or more of E; or one or more of D and one or more of F; or one or more of E and one or more of F; or one or more of all of D, E, and F.

International Patent Application Publication Nos. WO 2020/173991, WO 2020/173992, WO 2020/173993, WO 2020/173994, and WO 2020/173995 are incorporated herein by reference in their entirety.

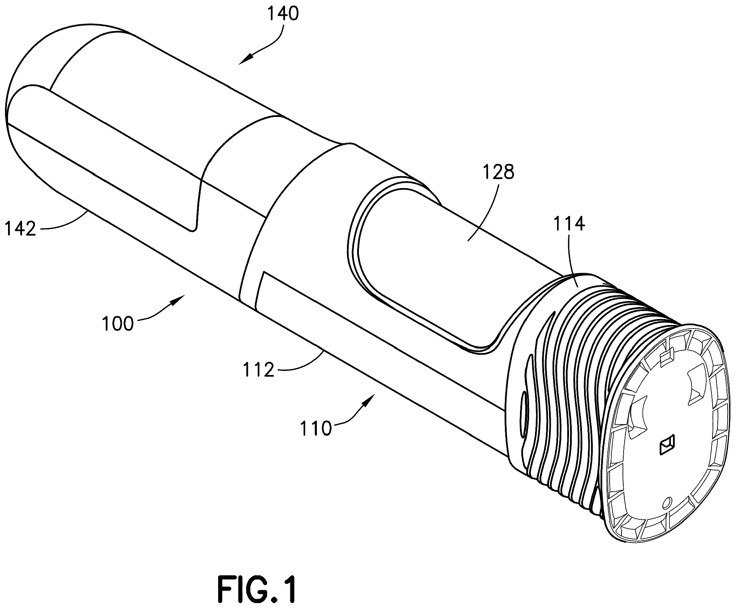

Turning to FIGS. 1-4, shown is a drug delivery device 100. The drug delivery device 100 includes a first subassembly 110, a second subassembly 140, and a syringe 116. The first subassembly 110 may include a lower housing shell 112, a cap 114, a syringe holder 118, a needle cover 124, and a cassette body 128. The second subassembly 140 may include a drive assembly including a plunger body 150. The plunger body 150 includes a plunger rod 152 and a spring guide member 154 including a drive member 156 and a drive opening 158. The second subassembly 140 may further include a motor body 159, a lever actuation member 130, and an upper housing shell 142. The syringe 116 may be received by the syringe holder 118 and includes a barrel 117, a stopper 122, a needle 120, and a rigid needle shield (RNS) 126. Although an RNS is utilized in the example shown in the accompanying drawings, other suitable needle shield arrangements may be utilized. The lower housing shell 112, the cassette body 128, and the upper housing shell 142 generally form a housing for receiving the various components of the drug delivery device 100, although other suitable housing arrangements may be utilized. The first subassembly 110 and the second subassembly 140 may be secured to each other during assembly by a locking clip, although other suitable arrangements may be utilized. The upper housing shell 142 includes an outer lip 143 configured to fit around an inner lip 113 of the lower housing shell 112 when the device 100 is assembled. The drug delivery device 100 may be an auto-injector, although the features described herein may be incorporated into other suitable drug delivery devices.

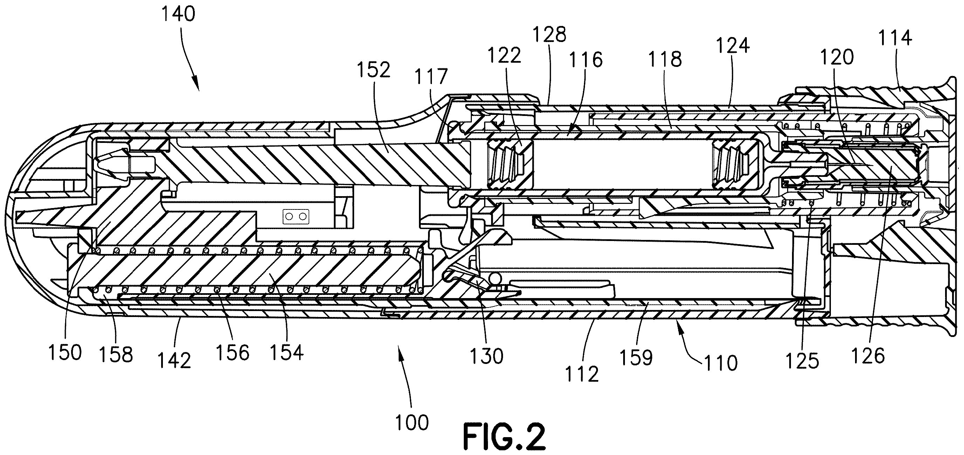

With continued reference to FIG. 2, the drive member 156 may be a compression spring received within a drive opening 158 defined by the plunger body 150, although other suitable drive members may be utilized, including, but not limited to, compressed gas, an electric motor, hydraulic pressure, other types of springs, etc. The drive member 156 engages the plunger body 150 and the motor body 159 and biases the plunger body 150 in a direction extending from the second subassembly 140 toward the first subassembly 110. The plunger body 150 defines a lever opening that receives the lever actuation member 130 and defines the rotation axis of the lever actuation member 130. The lever actuation member 130 prevents movement of the plunger body 150 when the lever actuation member 130 is in the locked position through engagement of the lever actuation member 130 with the motor body 159. Upon rotation of the lever actuation member 130 from the locked position to the released position, the lever actuation member 130 is disengaged from the motor body 159 thereby allowing the drive member 156 to move the plunger body 150 and the plunger rod 152 toward the first subassembly 110. The plunger rod 152 and the drive member 156 are spaced from and parallel to each other and extend in a longitudinal direction of the device 100.

The spring guide member 154 is secured to the upper housing shell 142 and received within the drive opening 158 of the plunger body 150. The drive member 156 is received by the spring guide member 154 such that the drive member 156 is positioned between the plunger body 150 and the spring guide member 154. In some embodiments, the drive assembly may also include a plunger rod cover (not shown) that receives the plunger rod 152 of the plunger body 150. The plunger rod cover may be configured to guide insertion of the plunger rod 152 into the barrel 117 of the syringe 116 and engage the stopper 122 to dispense the medicament from the barrel 117 of the syringe 116.

The drug delivery device 100 is configured to automatically deliver a dose of medicament from the syringe 116 to a patient upon actuation of the device 100. More specifically, upon actuation of the drug delivery device 100, the drive assembly is configured to engage the stopper 122 of the syringe 116, displace the syringe 116 such that the needle 120 pierces the skin of the patient, and displace the stopper 122 within the barrel 117 of the syringe 116 to deliver the medicament within the barrel 117. The drug delivery device 100 includes a storage position, a pre-use position, an actuation position, an injection position, and a post-use position, as described in International Patent Application Publication Nos. WO 2020/173991, WO 2020/173992, WO 2020/173993, WO 2020/173994, and WO 2020/173995.

The needle cover 124 is configured to shield the needle 120 of the syringe 116 from the patient when the device 100 is in the pre-use and the post-use positions. In particular, the needle cover 124 is moveable between a pre-use position, an actuation position, and a post-use positon, with a spring 125 biasing the needle cover 124 towards the pre-use position and the post-use position. The spring 125 may be positioned between the needle cover 124 and the syringe holder 118, although other suitable arrangements may be utilized.

The lever actuation member 130 is moveable between a locked position where movement of the drive assembly is prevented and a released position where movement of the drive assembly is allowed. More specifically, the lever actuation member 130 is rotatable about a rotation axis between the locked position and the released position. When the lever actuation member 130 is in the locked position, the lever actuation member 130 is engaged with the motor body 159 and the drive assembly to prevent movement of the drive assembly.

When the lever actuation member 130 is in the released position, which may be achieved by pressing the needle cover 124 onto the patient's skin at the site of injection, shifting the needle cover proximally into the lower housing shell 112, the lever actuation member 130 is disengaged from the motor body 159 thereby allowing movement of the drive assembly toward the syringe 116. The rotation axis of the lever actuation member 130 extends perpendicular to a longitudinal axis of the device 100, although other suitable arrangements may be utilized.

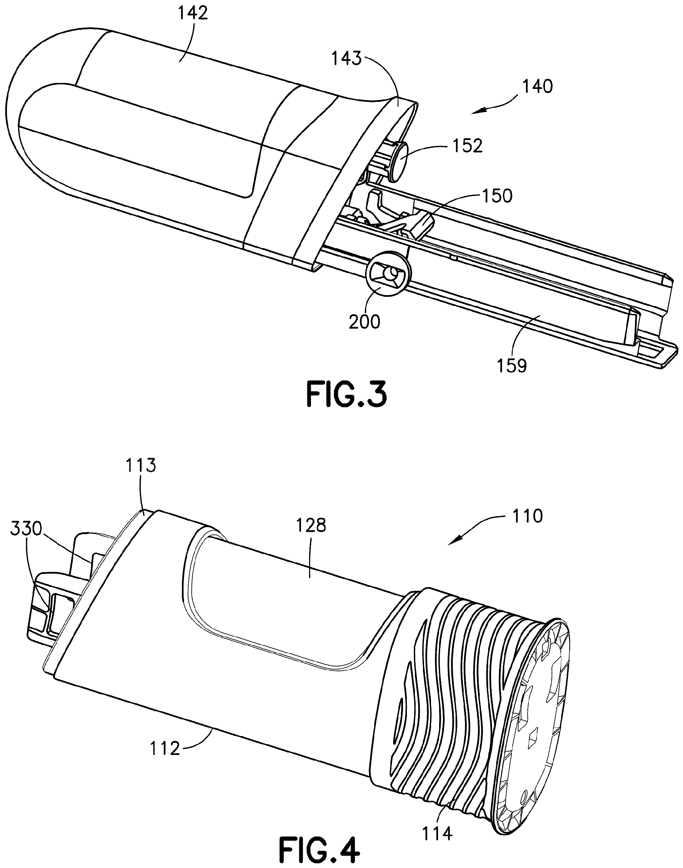

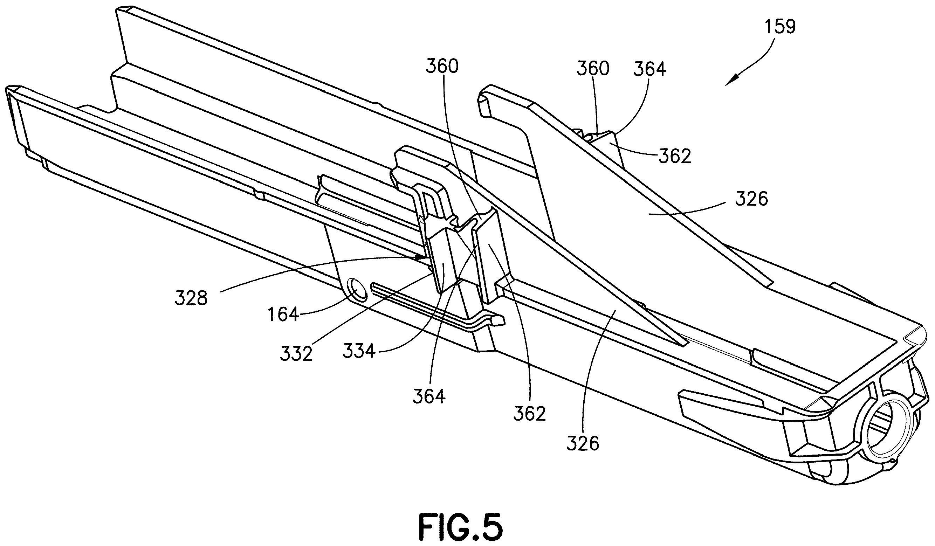

FIGS. 3 and 4 illustrate the second subassembly 140 and the first subassembly 110, respectively, as these subassemblies may be provided for final assembly of the device 100. As shown in FIG. 3, a safety pin 200 is inserted through apertures 164 (see FIG. 5) of the motor body 159. The apertures 164 are located in proximity to a distal end of the plunger body 150 so that with the safety pin 200 inserted through the apertures 164, the safety pin 200 prevents rotation of the lever actuation member 130 and unintentional actuation of the drive member 156. As will be described, the safety pin 200 is removed prior to or during final assembly of the device 100.

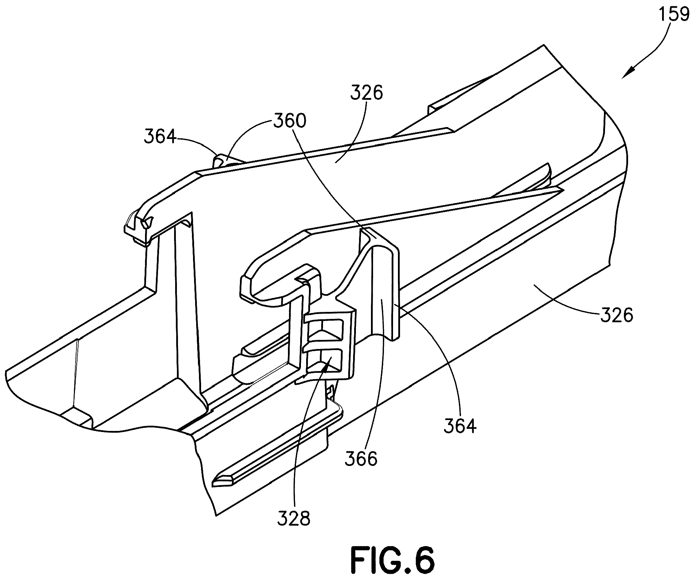

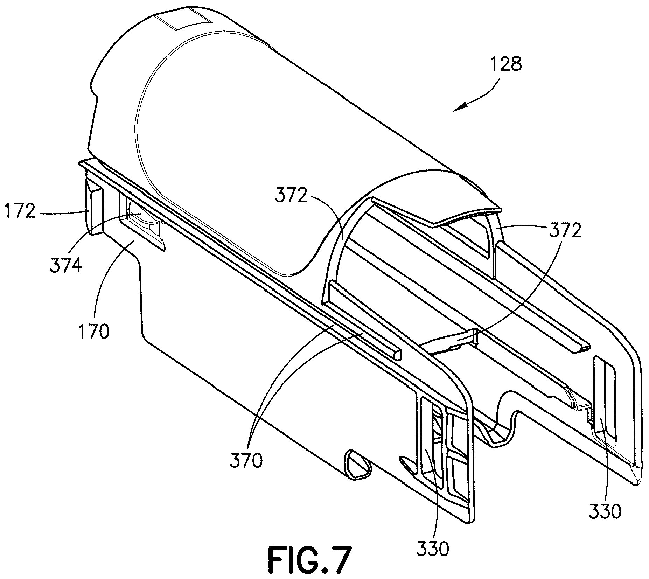

Referring now to FIGS. 5 and 6, the motor body 159 includes a pair of opposing sidewalls 326 configured to receive the plunger body 150 therebetween. The motor body 159 defines the safety pin apertures 164 extending transversely through the sidewalls 326 to facilitate insertion of the safety pin 200 (see FIG. 3). The motor body 159 further includes at least one cassette clip 328 extending from the respective sidewalls 326 for engaging complementary openings 330 (see FIGS. 7-8) defined by the cassette body 128. Engagement of the at least one cassette clip 328 with the openings 330 secures the motor body 159 to the cassette body 128, which is discussed in more detail below. The at least one cassette clip 328 includes an angled face 332 and a planar face 334, which are configured to allow insertion of the at least one cassette clip 328 into the corresponding openings 330 of the cassette body 128, but prevent easy removal of the cassette clips 328 once inserted into the openings 330 of the cassette body 128.

The motor body 159 is configured to be inserted in the upper housing shell 142 of the second subassembly 140. The motor body 159 includes at least one shell stop 360 extending outward from the opposing sidewalls 326 of the motor body 159. Each shell stop 360 has a planar proximal face 362 configured to engage an internal structure of the upper housing shell 142 to index the motor body 159 in the upper housing shell 142. The at least one shell stop 360 extends a sufficient distance outward from the sidewalls 326 of motor body 159 so that the at least one shell stop 360 remains at least partially engaged with the corresponding internal structure of the upper housing shell 142 when the sidewalls 326 of the motor body 159 are deflected inward during connection of the motor body 159 to the cassette body 128. In particular, each of the at least one shell stops 360 includes a cantilevered end 364. The cantilevered end 364 is reinforced by a radiused edge 366 to prevent failure of the shell stop 360 due to load applied to the cantilevered end 364. For example, the radiused edge 366 may be sized to prevent breakage of the at least one shell stop 360 if the device 100 is dropped during handling.

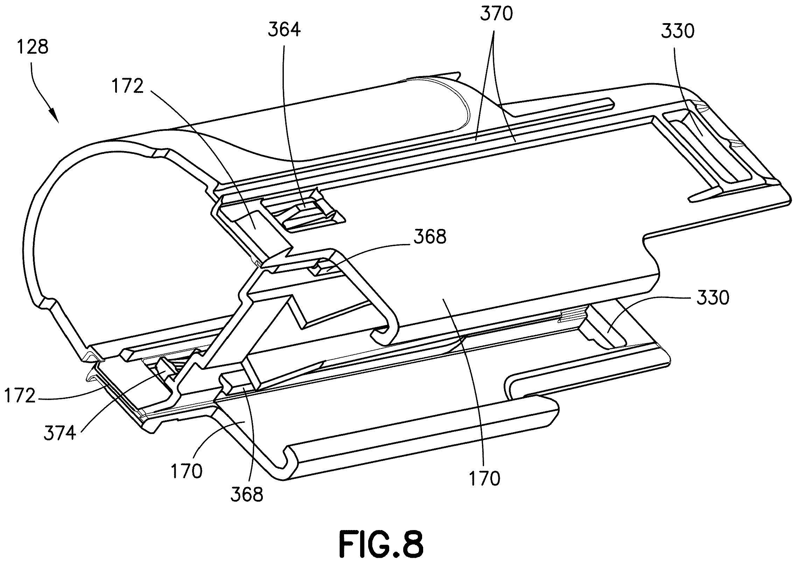

Referring to FIGS. 7 and 8, the cassette body 128 is generally U-shaped and configured to fit within the lower housing shell 112 and receive the syringe 116 and various other components as described herein in connection with FIGS. 1-4. The cassette body 128 includes the openings 330 that receive the corresponding cassette clips 328 of the motor body 159 when the device is assembled. The cassette body 128 includes a needle cover clip 374 that engage the needle cover 124 to restrict the axial movement of the needle cover 124 relative to the cassette body 128. The cassette body 128 further includes motor body ribs 368 and upper housing shell ribs 370, which are configured to engage corresponding portions of the motor body 159 and the lower housing shell 112, respectively, to aid in the assembly of the device 100. The cassette body 128 also includes syringe holder stops 372, which are configured to engage portions of the syringe holder 118 to limit the axial movement of the syringe holder 118 relative to the cassette body 128.

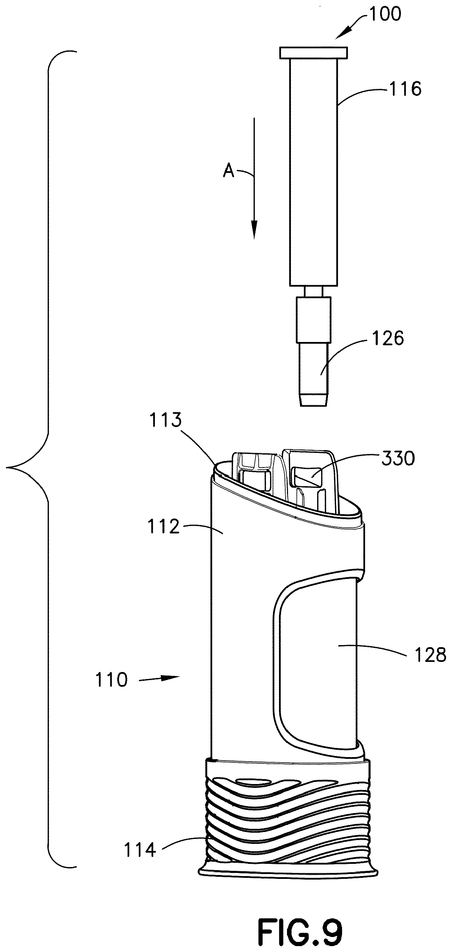

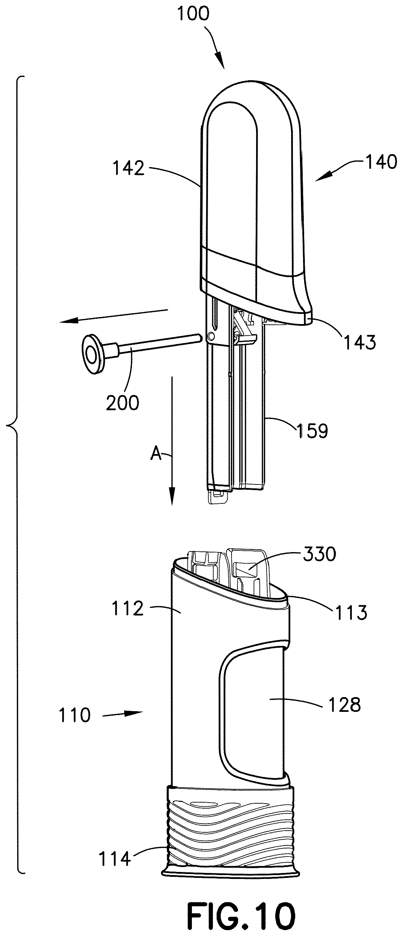

Referring now to FIGS. 9-11, the drug delivery device 100 may be provided for final assembly as three components, namely the first subassembly 110, the second subassembly 140, and the syringe 116. At final assembly, the syringe 116 may be inserted in a longitudinal direction A into the cassette body 128 of the first subassembly 110 until the syringe 116 is fully seated in the syringe holder 118, as shown in FIG. 9. Then, as shown in FIG. 10, the safety pin 200 may be removed from the motor body 159 to free the lever actuation member 130 in preparation for the connecting the first subassembly 110 to the second subassembly 140. In non-limiting embodiments, safety pin 200 is removed prior to introducing syringe 116 into first sub assembly 110. With safety pin 200 removed, the second subassembly 140, as a unit, may be brought into engagement with the first subassembly 110, again along the longitudinal direction A. As the second subassembly 140 is brought into engagement with the first subassembly 110, the cassette clips 328 of the motor body 159 engage the corresponding openings 330 of the cassette body 128. In particular, the angled face 332 of the cassette clips 328 deflect the cassette body 128 outward and/or deflect the sidewalls 326 of the motor body 159 inward as the cassette clips 328 contact the cassette body 128. Once the cassette clips 328 are aligned with the openings 330 of the cassette body 128, the motor body 159 and the cassette body 128 return to their respective undeflected states. Contemporaneously with or shortly after the cassette clips 328 snap into place in the openings 330, the outer lip 143 of the upper housing shell 142 engages the inner lip 113 of the lower housing shell 112, completing connection of the first subassembly 110 with the second subassembly 140.

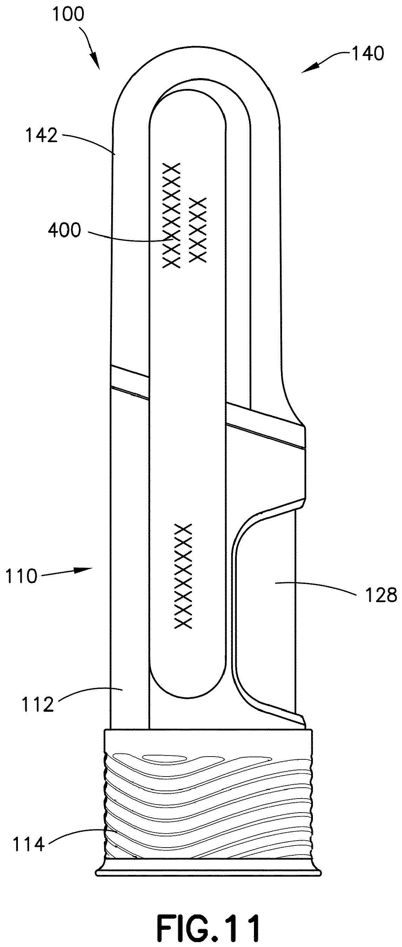

With continued reference to FIG. 11, once the first subassembly 110 and the second subassembly 140 have been connected, a label 400 can be applied to the device 100. The label 400 may span the first subassembly 110 and the second subassembly 140 so that first subassembly 110 and the second subassembly 140 cannot be separated without destroying the label 400.

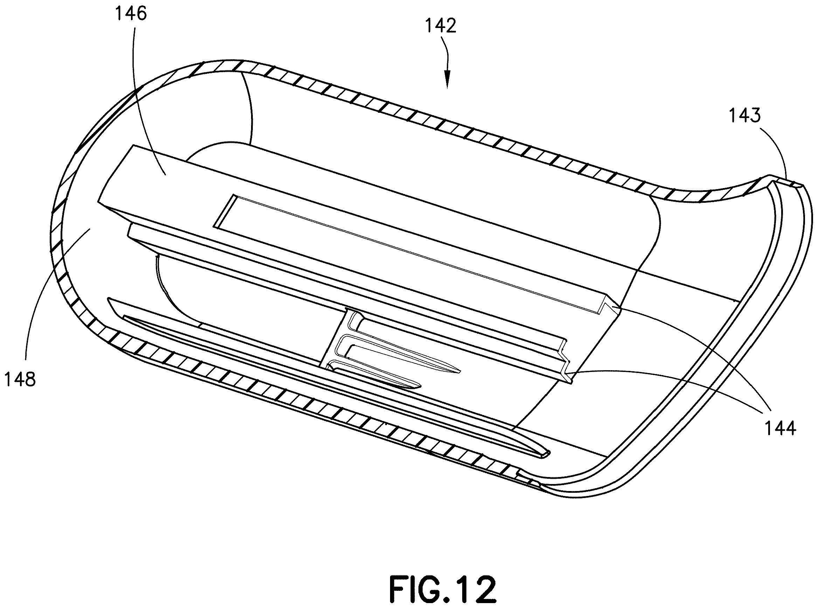

Referring now to FIG. 12, each side of the upper housing shell 142 includes a pair of spaced-apart reinforcing ribs 144 extending along a longitudinal axis of the upper housing shell 142 and inward toward a central axis of the upper housing shell 142. The reinforcing ribs 144 are configured to increase rigidity of the upper housing shell 142 and may also facilitate alignment of the internal components of the second subassembly 140 during assembly. Each of the reinforcing ribs 144 may have an L-shaped, Z-shaped, or stepped profile, though other profiles are also within the scope of the present disclosure. A connecting wall 146 may extend between and connect the reinforcing ribs 144 to further increase rigidity of the upper housing shell 142. The connecting wall 146 may further attach to and extend from a proximal end 148 of the upper housing shell 142 and extend at least partially along the reinforcing ribs 144. The connecting wall 146 may terminate at a location between the proximal end 148 of the upper housing shell 142 and distal ends of the reinforcing ribs 144.

Although the present disclosure has been described in detail for the purpose of illustration based on what is currently considered to be the most practical and preferred embodiments, it is to be understood that such detail is solely for that purpose and that the present disclosure is not limited to the disclosed embodiments, but, on the contrary, is intended to cover modifications and equivalent arrangements that are within the spirit and scope of the appended claims. For example, it is to be understood that the present disclosure contemplates that, to the extent possible, one or more features of any embodiment can be combined with one or more features of any other embodiment.

## Claims

1. A drug delivery device comprising:
an upper housing shell;
a lower housing shell;
a syringe comprising a barrel, a stopper, and a needle, at least a portion of the syringe positioned within the lower housing shell;
a drive assembly comprising a plunger body configured to move the stopper within the barrel upon actuation of the drive assembly;
a cassette body configured to receive the syringe; and
a motor body configured to engage the cassette body,
wherein the motor body comprises:
a pair of sidewalls; and
at least one shell stop extending outward from the sidewalls and configured to engage an internal structure of the upper housing shell.

2. The drug delivery device of claim 1, wherein the at least one shell stop comprises a planar proximal face.

3. The drug delivery device of claim 1 or 2, wherein the at least one shell stop comprises a cantilevered end.

4. The drug delivery device of claim 3, wherein the at least one shell stop comprises a radiused edge reinforcing the cantilevered end.

5. The drug delivery device of any of claims 1-4, wherein sidewalls of the motor body are configured to deflect inward during connection of the motor body to the cassette body, and
wherein the at least one shell stop extends a sufficient distance outward from the sidewalls so that the at least one shell stop remains at least partially engaged with the internal structure of the upper housing shell when the sidewalls are deflected inward during connection of the motor body to the cassette body.

6. The drug delivery device of any of claims 1-5, wherein the upper housing shell comprises a pair of spaced apart reinforcing ribs extending along a longitudinal axis of the upper housing shell.

7. The drug delivery device of claim 6, wherein the reinforcing ribs have an L-shaped, Z-shaped, or stepped profile.

8. The drug delivery device of claim 6 or 7, wherein the upper housing shell comprises a connecting wall extending between and connecting the reinforcing ribs.

9. The drug delivery device of claim 8, wherein the connecting wall extends from a proximal end of the upper housing shell.

10. The drug delivery device of claim 8 or 9, wherein the connecting wall terminates at a location between the proximal end of the upper housing shell and distal ends of the reinforcing ribs.

11. The drug delivery device of any of claims 1-10, further comprising:
a first subassembly comprising:
the lower housing shell; and
the cassette body; and
a second subassembly comprising:
the upper housing shell; and
the motor body,
wherein the second subassembly is configured to be brought into engagement with the first subassembly to assemble the drug delivery device.

12. The drug delivery device of any of claims 1-11, wherein the lower housing shell comprises an inner lip, and
wherein the upper housing shell comprises an outer lip configured to engage the inner lip.

13. A drug delivery device comprising:
an upper housing shell;
a lower housing shell;
a syringe comprising a barrel, a stopper, and a needle, at least a portion of the syringe positioned within the lower housing shell;
a drive assembly comprising a plunger body configured to move the stopper within the barrel upon actuation of the drive assembly;
a cassette body configured to receive the syringe; and
a motor body configured to engage the cassette body,
wherein the upper housing shell comprises:
a pair of spaced apart reinforcing ribs extending along a longitudinal axis of the upper housing shell; and
a connecting wall extending between and connecting the reinforcing ribs.

14. The drug delivery device of claim 13, wherein the connecting wall extends from a proximal end of the upper housing shell.

15. The drug delivery device claim 13 or 14, wherein the connecting wall terminates at a location between the proximal end of the upper housing shell and distal ends of the reinforcing ribs.

16. A method of assembling a drug delivery device, the method comprising:
inserting a syringe into a first subassembly, the first subassembly comprising a cassette body;
removing a safety pin from a motor body of a second subassembly; and
bringing the second subassembly into engagement with the first subassembly such that:
at least one cassette clip of the motor body engages at least one opening of the cassette body; and
an outer lip of the second subassembly engages an inner lip of the first subassembly.

17. The method of claim 16, further comprising applying a label to the first subassembly and the second subassembly.

18. The method of claim 16 or 17, wherein the motor body comprises a pair of sidewalls and at least one shell stop extending outward from the sidewalls, wherein the at least one shell stop is configured to engage an upper housing shell of the second subassembly.

19. The method of claim 18, wherein the at least one shell stop includes a cantilevered end and a radiused edge.

20. The method of any of claims 15-19, wherein an upper housing shell of the second subassembly comprises:
a pair of spaced-apart reinforcing ribs; and
a connecting wall extending between and connecting the reinforcing ribs.
